# EUROPÄISCHE PATENTSCHRIFT

(11) **EP 3 659 406 B1**
(45) Veröffentlichungstag und Bekanntmachung des Hinweises auf die Patenterteilung: **25.01.2023**
(21) Anmeldenummer: 18745576.1
(22) Anmeldetag: 23.07.2018
(51) Int. Cl.: H05H 1/24, A61N 1/44, A61L 2/14

(54) **ELEKTRODENANORDNUNG FÜR EINE DIELEKTRISCH BEHINDERTE PLASMABEHANDLUNG**
ELECTRODE ARRAY FOR A DIELECTRICALLY IMPEDED PLASMA TREATMENT
AGENCEMENT D'ÉLECTRODES POUR UN TRAITEMENT PAR PLASMA DIÉLECTRIQUEMENT ENTRAVÉ

(30) Priorität: 25.07.2017 DE 102017116800
(43) Veröffentlichungstag der Anmeldung: 03.06.2020
(73) Patentinhaber: Cinogy GmbH, 37115 Duderstadt (DE)
(72) Erfinder: WANDKE, Dirk, 37308 Heiligenstadt (DE); HAHNL, Mirko, 37339 Berlingerode (DE); STORCK, Karl-Otto, 37115 Duderstadt (DE); TRUTWIG, Leonhard, 37115 Duderstadt/Gerblingerode (DE); RICKE, Melanie, 37191 Katlenburg-Lindau (DE)
(74) Vertreter: Gramm, Lins & Partner Patent- und Rechtsanwälte PartGmbB
(86) Internationale Anmeldenummer: PCT/EP2018/069929
(87) Internationale Veröffentlichungsnummer: WO 2019/020569

(56) Entgegenhaltungen:
- WO-A2-2007/011865
- CN-A- 106 668 897
- DE-A1-102008 030 913
- DE-A1-102009 060 627
- DE-A1-102013 019 057
- DE-A1-102014 220 488
- KR-B1- 101 407 672
- US-A1- 2013 345 620

## Beschreibung

Die Erfindung betrifft eine Elektrodenanordnung für eine dielektrisch behinderte Plasmabehandlung einer Oberfläche eines Körpers, mit mindestens einer flexiblen flächigen Elektrode und einem Dielektrikum aus einem flächigen flexiblen ersten Material, das mit einer einen direkten Stromfluss verhindernden Schicht die Elektrode von der zu behandelnden Oberfläche abschirmt, wobei das Dielektrikum über eine Struktur mit Vorsprüngen auf der zu behandelnden Oberfläche aufliegen kann und dabei zwischen den Vorsprüngen Lufträume für die Ausbildung des Plasmas ausgebildet sind, die eine zur behandelnden Oberfläche offene Seite und einen bodenseitigen Abschluss durch die den direkten Stromfluss verhindernde Schicht des Dielektrikums aufweisen.

Die mindestens eine Elektrode der Elektrodenanordnung ist mit einer für die Plasmaerzeugung benötigten Hochspannung, die vorzugsweise als Wechselspannung verwendet wird, verbindbar.

Die Elektrodenanordnung ist dazu eingerichtet, dass als Gegenelektrode die zu behandelnde Oberfläche des Körpers verwendet wird. Hierzu muss der Körper ein elektrisch leitender Körper sein, beispielsweise ein menschlicher oder tierischer Körper, dessen Hautoberfläche behandelt werden soll, oder ein anderer elektrisch leitender Körper.

Zu diesem Zweck ist es beispielsweise möglich, nur eine einzige Elektrode für die Generierung des Plasmas zu verwenden und die Oberfläche bzw. den Körper als Gegenelektrode (Masse) zu verwenden. Hierdurch wird vorteilhaft eine große Behandlungstiefe innerhalb des Körpers erreicht.

Es ist ferner beispielsweise möglich, wenigstens zwei Elektroden in der Elektrodenanordnung vorzusehen, die mit demselben Hochspannungspotential beaufschlagt werden, sodass die zu behandelnde Oberfläche, auf der die Elektrodenanordnung aufliegt, als Gegenelektrode für die Plasmabildung fungiert. Alternativ ist es beispielsweise möglich, die beiden Elektroden gegenphasig mit der Wechsel-Hochspannung zu versorgen, wobei die zu behandelnde Oberfläche wiederum als Gegenelektrode fungiert.

Unter "Lufträumen" werden im Rahmen dieser Anmeldung Leerräume verstanden, die üblicherweise mit Luft gefüllt sind, aber auch für bestimmte Anwendungsfälle mit einem geeigneten Gas gefüllt werden können, um spezielle Plasmen auszubilden.

Für eine Elektrodenanordnung der eingangs genannten Art ist es wesentlich, dass das Dielektrikum eine durchgehende Schicht bildet, mit der die Elektrode von der zu behandelnden Oberfläche abgeschirmt ist. Diese Schicht verhindert einen direkten oder galvanischen Stromfluss zwischen der Elektrode und der zu behandelnden Oberfläche und ist eine einen direkten Stromfluss verhindernde Schicht im Sinne dieser Anmeldung.

Eine Elektrodenanordnung der eingangs genannten Art ist durch DE 10 2009 060 627 B4 bekannt. Deren Aufbau ermöglicht, dass die Elektrodenanordnung auch auf unregelmäßig gewölbte Oberflächen zur Durchführung einer Plasmabehandlung aufgelegt werden kann. Um die für die Ausbildung eines Plasmas notwendigen Lufträume zwischen der zu behandelnden Oberfläche und der einen direkten Stromfluss verhindernden Schicht des Dielektrikums ausbilden zu können, ist dabei vorgesehen, dass das Dielektrikum auf seiner zu der zu behandelnden Oberfläche zeigenden Seite mit einer Struktur versehen ist, die Vorsprünge aufweist. Die Struktur besteht dabei bspw. aus vorstehenden Noppen, die luftdurchlässige Zwischenräume ausbilden.

Eine weitere Elektrodenanordnung der eingangs genannten Art ist aus DE 10 2015 117 715 A1 bekannt. Auch diese Elektrodenanordnung ist mit einer Struktur ausgebildet, damit ein Plasma entstehen kann, wenn die Elektrodenanordnung auf der Oberfläche aufliegt. Die Struktur ist dabei eine Gitterstruktur aus aneinander angrenzenden Wänden, die zahlreiche, die Lufträume ausbildende Kammern begrenzen, welche einen bodenseitigen Abschluss durch die den direkten Stromfluss verhindernde Schicht des Dielektrikums sowie eine zur behandelnden Oberfläche offene Seite aufweisen. Die Kammern können dabei bspw. einen quadratischen, runden, ovalen oder vieleckigen Querschnitt haben.

Die bekannten Elektrodenanordnungen haben sich bewährt und sind insbesondere auch für die Behandlung der Hautoberfläche eines menschlichen oder tierischen Körpers geeignet. Durch die Plasmabehandlung können therapeutische oder kosmetische Wirkstoffe verbessert aufgenommen werden, sodass die Plasmabehandlung die angestrebte therapeutische oder kosmetische Wirkung verstärkt. Darüber hinaus sorgt die Plasmabehandlung für eine wirksame Keimreduktion, da sie Mikroorganismen zerstört und insbesondere eine bakterizide und fungizide Wirkung auf der Haut ausübt. Weiterhin führt die Plasmabehandlung zu einer Erhöhung der Mikrozirkulation im Gewebe.

Damit eine derartige Behandlung auch auf unregelmäßig dreidimensional geformten Oberflächen möglich ist, müssen sowohl die Elektrode als auch das Dielektrikum und die Struktur, mit der die Elektrodenanordnung auf der zu behandelnden Oberfläche aufliegt, flexibel ausgebildet sein. Um eine möglichst gute Anpassung der Elektrodenanordnung an die zu behandelnde Oberfläche zu erreichen, ist es dabei wünschenswert, dass die Bestandteile der Elektrodenanordnung und insbesondere das Dielektrikum der Elektrodenanordnung aus einem möglichst flexiblen Material bestehen. Bei der Behandlung von menschlichen oder tierischen Körpern bietet dies zudem den Vorteil, dass die Elektrodenanordnung besonders komfortabel und angenehm auf dem Körper getragen werden kann.

Gleichzeitig muss allerdings sichergestellt werden, dass ein vorgegebener Abstand zwischen der zu behandelnden Oberfläche und der einen direkten Stromfluss verhindernden Schicht des Dielektrikums eingehalten wird, um die gewünschte Ausbildung des Plasmas in diesem Zwischenraum und damit eine effektive Plasmabehandlung zu gewährleisten. Bei den aus dem Stand der Technik bekannten Elektrodenanordnungen muss somit sichergestellt werden, dass die durch die Struktur und ihre Vorsprünge ausgebildeten Lufträume während der Behandlung eine vorgegebene Größe und insbesondere eine vorgegebene Höhe aufweisen.

Durch diese Anforderung sind bei den aus dem Stand der Technik bekannten Elektrodenanordnungen der Auswahl des flexiblen Materials für die Elektrodenanordnung und insbesondere für das Dielektrikum der Elektrodenanordnung physikalische Grenzen gesetzt. Wird hierfür ein Material ausgewählt, das eine zu hohe Flexibilität aufweist, so können die vorgegebene Höhe der Lufträume und damit der vorgegebene Abstand zwischen der zu behandelnden Oberfläche und der einen direkten Stromfluss verhindernden Schicht des Dielektrikums nicht mehr sichergestellt werden. Dies liegt darin begründet, dass die Vorsprünge der die Lufträume ausbildenden Struktur in diesem Fall nicht die notwendige Steifigkeit aufweisen und sich während der Behandlung in unerwünschtem Maße verformen. Dadurch wird die Effektivität der Plasmabehandlung verringert oder eine wirksame Plasmabehandlung sogar ganz verhindert.

Der vorliegenden Erfindung liegt daher die Problemstellung zugrunde, die bewährten vorbekannten Elektrodenanordnungen unter Beibehaltung ihrer Vorteile dahingehend zu verbessern, dass sie sich in ihrer Form noch besser an die zu behandelnde Oberfläche anpassen und zugleich eine wirksame Plasmabehandlung ermöglichen.

Zur Lösung dieser Aufgabe ist eine Elektrodenanordnung der eingangs erwähnten Art erfindungsgemäß dadurch gekennzeichnet, dass die Struktur eine Vielzahl von Distanzelementen aus einem zweiten Material, dessen Flexibilität geringer ist als die Flexibilität des ersten Materials, aufweist, wobei die Vorsprünge der Struktur teilweise oder vollständig durch die Distanzelemente gebildet sind.

Die Distanzelemente stellen dabei in vorteilhafter Weise einen definierten Abstand zwischen der zu behandelnden Oberfläche und der einen direkten Stromfluss verhindernden Schicht des Dielektrikums sicher. Zu diesem Zweck sind die Distanzelemente aus einem zweiten Material gefertigt, dessen Flexibilität geringer ist als die Flexibilität des ersten Materials, aus dem das Dielektrikum der Elektrodenanordnung gefertigt ist. Auf diese Weise ermöglicht die erfindungsgemäße Elektrodenanordnung, dass ihr Dielektrikum aus einem besonders flexiblen, bei den vorbekannten Elektrodenanordnungen aus den zuvor erläuterten Gründen nicht verwendbaren Material gefertigt werden kann, um eine verbesserte Anpassung der Elektrodenanordnung an die zu behandelnde Oberfläche unter Beibehaltung des erforderlichen Abstands zwischen der zu behandelnden Oberfläche und der einen direkten Stromfluss verhindernden Schicht zu erreichen.

Eine geringere Flexibilität des Materials kann bspw. dadurch erreicht werden, dass ein Material verwendet wird, das eine größere Härte, insbesondere eine größere Shore-Härte, aufweist. Eine geringere Flexibilität des Materials kann auch dadurch erreicht werden, dass ein elastisches Material mit einer geringeren Elastizität verwendet wird. Die Elastizität des Materials kann dabei bspw. durch sein Elastizitätsmodul und/oder seinen Elastizitätstensor quantifiziert werden.

Geeignete Materialien für die Fertigung des flexiblen Dielektrikums sind bspw. flexible Silikone, insbesondere Silikonkautschuke.

Mit der erfindungsgemäßen Elektrodenanordnung ist es bspw. möglich, das Dielektrikum der Elektrodenanordnung aus einem flexiblen Silikon mit einer Shore-Härte zu fertigen, die kleiner ist als die kleinste Shore-Härte der für die Fertigung der vorbekannten Elektrodenanordnungen verwendbaren Silikone. Bspw. ist es möglich, für die Fertigung des Dielektrikums ein Silikon mit einer Härte zwischen 10 Shore und 40 Shore, vorzugsweise zwischen 15 und 25 Shore, insbesondere 20 Shore, zu verwenden. Die in der vorliegenden Anmeldung angegebenen Shore-Härten bezeichnen dabei Shore-Härten nach Shore-A.

Durch die Distanzelemente der erfindungsgemäßen Elektrodenanordnung kann vorteilhaft eine unerwünschte Verformung der Vorsprünge der Struktur während der Behandlung vermieden und so eine Einhaltung des notwendigen Abstands zwischen der zu behandelnden Oberfläche und der durchgehenden, den direkten Stromfluss von der Elektrode verhindernden Schicht des Dielektrikums sichergestellt werden. Die Höhe der Distanzelemente bestimmt dabei im Wesentlichen den Abstand zwischen der zu behandelnden Oberfläche und der den direkten Stromfluss verhindernden Schicht des Dielektrikums und damit die Höhe der Lufträume für die Ausbildung des Plasmas. Vorteilhaft kann die Höhe der Distanzelemente zu diesem Zweck bspw. zwischen 0,05 mm und 3,0 mm betragen.

Vorteilhaft ist es bspw. möglich, die Distanzelemente aus einem stabilen Kunststoff zu fertigen, der eine geringere Flexibilität aufweist als das für das Dielektrikum verwendete Material, wobei letzteres bspw. ein flexibles Silikon sein kann. Vorteilhaft ist es bspw. auch möglich, sowohl das Dielektrikum als auch die Distanzelemente der erfindungsgemäßen Elektrodenanordnung aus einem flexiblen Silikon der zuvor genannten Art zu fertigen, wobei für die Distanzelemente ein Silikon mit einer größeren Shore-Härte als für das Dielektrikum verwendet wird. Das für die Distanzelemente verwendete Material ist allerdings grundsätzlich beliebig, es muss lediglich eine geringere Flexibilität aufweisen als das Material des Dielektrikums.

Die Vorsprünge der Struktur können vollständig durch die Distanzelemente gebildet sein. In diesem Fall bestehen die Vorsprünge allein aus den Distanzelementen. Die Vorsprünge der Struktur können aber auch nur teilweise durch die Distanzelemente gebildet sein. In diesem Fall sind die Distanzelemente zwar Bestandteile der Vorsprünge, die Vorsprünge weisen allerdings neben den Distanzelementen noch einen oder mehrere andere Bestandteile auf.

Durch die erfindungsgemäße Elektrodenanordnung kann in vorteilhafter Weise erreicht werden, dass die Struktur in der Fläche eine deutlich höhere Flexibilität aufweist als in der Höhe. Insbesondere kann dabei vorteilhaft erreicht werden, dass die Struktur - und damit die Elektrodenanordnung - eine geringe Biegesteifigkeit und/oder eine geringe Torsionssteifigkeit aufweist, um ihre Form gut an die zu behandelnde Oberfläche anpassen zu können, während die Struktur zugleich eine große Dehnsteifigkeit, also eine große Stabilität, gegenüber Kräften aufweist, die im Wesentlichen senkrecht zu der zu behandelnden Oberfläche einwirken, sodass die Erhaltung der Lufträume sichergestellt werden kann.

In einer vorteilhaften Weiterbildung der Erfindung sind die Distanzelemente über Verbindungsabschnitte, die eine geringere Biegesteifigkeit und/oder eine geringere Torsionssteifigkeit aufweisen als die Distanzelemente, miteinander verbunden.

Vorteilhaft können die Verbindungsabschnitte dabei insbesondere Verbindungsabschnitte aus dem ersten Material oder einem dritten Material, dessen Flexibilität größer ist als die Flexibilität des zweiten Materials, sein. Die geringere Steifigkeit der Verbindungsabschnitte im Vergleich zu den Distanzelementen wird in diesem Fall durch die größere Flexibilität des Materials der Verbindungsabschnitte im Vergleich zu dem Material der Distanzelemente erreicht.

Alternativ oder ergänzend hierzu kann die Form der Verbindungsabschnitte so ausgebildet sein, dass die Verbindungsabschnitte eine geringere Steifigkeit aufweisen als die Distanzelemente. Hier können die Verbindungsabschnitte zwischen den Distanzelementen im Vergleich zu den Distanzelementen insbesondere dünn oder schmal ausgebildet sein, insbesondere eine geringere Breite und/oder eine geringere Höhe aufweisen als die Distanzelemente.

Die Verbindungsabschnitte zwischen den Distanzelementen können dabei beispielsweise durch das Dielektrikum der Elektrodenanordnung, insbesondere durch die einen direkten Stromfluss verhindernde Schicht des Dielektrikums, ausgebildet werden. Die Distanzelemente können zu diesem Zweck beispielsweise mit der der zu behandelnden Oberfläche zugewandten Seite des Dielektrikums, insbesondere mit der der zu behandelnden Oberfläche zugewandten Seite der einen direkten Stromfluss verhindernden Schicht des Dielektrikums, verbunden sein. Da das erste Material des Dielektrikums eine größere Flexibilität aufweist als das zweite Material der Distanzelemente, kann auf diese Weise besonders einfach erreicht werden, dass die Verbindungsabschnitte eine geringere Steifigkeit aufweisen als die Distanzelemente.

In einer weiteren vorteilhaften Weiterbildung der Erfindung ist vorgesehen, dass die Distanzelemente durch Verbindungsstege miteinander verbunden sind und auf diese Weise ein Distanzgitter ausbilden.

Vorteilhaft ist die Form der Verbindungsstege dabei so ausgebildet, dass die Verbindungsstege eine geringere Steifigkeit aufweisen als die Distanzelemente. Zu diesem Zweck können die Verbindungsstege zwischen den Distanzelementen im Vergleich zu den Distanzelementen insbesondere dünn und/oder schmal ausgebildet sein. Hierzu können die Verbindungsstege insbesondere eine geringere Breite und/oder eine geringere Höhe aufweisen als die Distanzelemente.

Dadurch kann vorteilhaft erreicht werden, dass das Distanzgitter in der Fläche eine deutlich höhere Flexibilität aufweist als in der Höhe. Insbesondere kann dabei vorteilhaft erreicht werden, dass das Distanzgitter eine geringe Biegesteifigkeit und/oder eine geringe Torsionssteifigkeit aufweist, um seine Form gut an die zu behandelnde Oberfläche anpassen zu können, während das Distanzgitter zugleich eine große Dehnsteifigkeit gegenüber Kräften aufweist, die im Wesentlichen senkrecht zu der zu behandelnden Oberfläche einwirken, d. h. gegenüber diesen Kräfte vergleichsweise stabil ist, sodass die Erhaltung der Lufträume sichergestellt werden kann.

Die Verbindungsstege des Distanzgitters können dabei vorteilhaft aus demselben Material wie die Distanzelemente selbst, d. h. aus dem zweiten Material, bestehen.

Alternativ hierzu können die Verbindungsstege aber auch aus einem anderen Material bestehen. Das Material der Verbindungsstege kann dabei insbesondere ein Material sein, dessen Flexibilität größer ist als die Flexibilität des Materials der Distanzelemente. Dadurch kann vorteilhaft die Steifigkeit der Verbindungsstege weiter verringert werden.

Eine solche Ausführungsform der erfindungsgemäßen Elektrodenanordnung, bei der die Distanzelemente ein Distanzgitter ausbilden, indem sie durch Verbindungsstege miteinander verbunden sind, bietet den erfindungsgemäßen Vorteil, dass die Stabilität der Struktur und ihrer Vorsprünge und damit der zwischen den Vorsprüngen ausgebildeten Lufträume verbessert werden kann.

In einer weiteren vorteilhaften Weiterbildung der Erfindung ist vorgesehen, dass das Distanzgitter einstückig ausgebildet ist. Dies bietet den Vorteil, dass das Distanzgitter besonders einfach und kostengünstig gefertigt werden kann, bspw. im Gießverfahren oder im 3D-Druckverfahren.

In einer weiteren vorteilhaften Weiterbildung der Erfindung sind die Distanzelemente im Inneren der Vorsprünge eingebettet, insbesondere vollständig im Inneren der Vorsprünge eingebettet, um die Vorsprünge zu verstärken. In diesem Fall werden die Vorsprünge der Struktur somit lediglich teilweise durch die Distanzelemente gebildet. Die Distanzelemente wirken dabei als Verstärkungen der Vorsprünge und erhöhen insbesondere die Steifigkeit der Vorsprünge. Die Distanzelemente können insbesondere vollständig eingebettet, d.h. allseitig umschlossen sein.

Eine solche Ausführungsform bietet den Vorteil, dass die Auflagefläche der Elektrodenanordnung auf der zu behandelnden Oberfläche nicht notwendigerweise durch die Distanzelemente gebildet wird. Die Struktur kann dabei aus einem Material mit besonders großer Flexibilität, insbesondere besonders geringer Härte, gefertigt sein, in das die Distanzelemente, die aus einem Material geringerer Flexibilität, insbesondere größerer Härte, gefertigt sind, eingebettet sind. Die Auflagefläche auf der zu behandelnden Oberfläche wird so durch das flexiblere Material der Struktur gebildet. Dadurch kann vorteilhaft zum einen ein fester Halt der Elektrodenanordnung auf der zu behandelnden Oberfläche sowie eine besonders gute Anpassung der Form der Elektrodenanordnung an die zu behandelnde Oberfläche erreicht werden. Zum anderen kann dadurch vorteilhaft erreicht werden, dass die Elektrodenanordnung auf der Oberfläche eines menschlichen oder tierischen Körpers als besonders angenehm wahrgenommen wird.

In einer weiteren vorteilhaften Weiterbildung der Erfindung ist vorgesehen, dass die Vorsprünge der Struktur aus den Distanzelementen und dem Dielektrikum gebildet sind, wobei die Distanzelemente in das die Distanzelemente umgebende Dielektrikum eingebettet, insbesondere vollständig eingebettet, sind.

Die Distanzelemente sind in dieser Ausführungsform somit zumindest teilweise, vorzugsweise vollständig, in das Dielektrikum eingebettet, welches die Distanzelemente zumindest teilweise, vorzugsweise vollständig, umgibt. Da das Material des Dielektrikums erfindungsgemäß eine größere Flexibilität aufweist als das Material der Distanzelemente, können mit dieser Ausführungsform auf besonders einfache Weise die in Zusammenhang mit der zuvor beschriebenen vorteilhaften Weiterbildung genannten Vorteile realisiert werden.

Vorteilhaft kann dabei der die Distanzelemente umgebende Teil der Struktur, insbesondere der die Distanzelemente umgebende Teil des Dielektrikums, für eine Anlage auf der Haut eines Lebewesens ausgebildet sein.

In einer weiteren vorteilhaften Weiterbildung der Erfindung ist vorgesehen, dass das die Distanzelemente umgebende Dielektrikum einstückig mit der den direkten Stromfluss verhindernden Schicht des Dielektrikums ausgebildet ist.

Es wird somit vorgeschlagen, dass das die Distanzelemente umgebende Dielektrikum und die den direkten Stromfluss verhindernde Schicht des Dielektrikums einstückig gefertigt sind. Vorteilhaft kann insbesondere das gesamte Dielektrikum einschließlich des die Distanzelemente umgebenden Teils des Dielektrikums einstückig ausgebildet, d.h. einstückig gefertigt sein.

Diese Ausführungsformen der erfindungsgemäßen Elektrodenanordnung bieten den Vorteil, dass die Elektrodenanordnung besonders einfach und kostengünstig im Gießverfahren herstellbar ist. Dabei können die Distanzelemente einfach in das Dielektrikum eingegossen werden. Ebenso ist vorteilhaft aber auch der schnelle Aufbau nach Art eines Prototyps im 3D-Druckverfahren möglich.

Alternativ ist es aber auch denkbar, dass der die Distanzelemente umgebende Teil der Struktur, insbesondere der die Distanzelemente umgebende Teil des Dielektrikums, als separates Teil gefertigt wird, um dann an die den direkten Stromfluss verhindernde Schicht des Dielektrikums angesetzt zu werden. Die Herstellung einer festen Verbindung kann dabei in üblicher Weise, also mechanisch in einer Gehäusestruktur, formschlüssig und/oder stoffschlüssig, insbesondere durch Kleben oder Schweißen, erfolgen. Auch hier ist es möglich, dass die Distanzelemente in die Struktur eingegossen werden.

Eine solche Form einer separat hergestellten Struktur bietet den Vorteil, dass insbesondere bei der Reinigung von Wunden eine leichte Austauschbarkeit des mit der Wunde in Berührung gelangenden Teils der Elektrodenanordnung ermöglicht werden kann. Zu diesem Zweck kann die separate Struktur einschließlich der Distanzelemente als entfernbares Einmalteil verwendet oder auch wegen ihres geringen Volumens einfach sterilisiert werden.

Die Distanzelemente als solche können vorteilhaft ebenfalls in entsprechender Weise im Gießverfahren oder im 3D-Druckverfahren gefertigt werden.

In einer weiteren vorteilhaften Weiterbildung der Erfindung ist vorgesehen, dass die Vorsprünge der Struktur vollständig durch die Distanzelemente gebildet sind. Die Distanzelemente sind dabei auf ihrer der Elektrode zugewandten Seite mit der einen direkten Stromfluss verhindernden Schicht verbunden und bilden auf ihrer der zu behandelnden Oberfläche zugewandten Seite eine Anlagefläche an der zu behandelnden Oberfläche.

In dieser Ausführungsform werden die Vorsprünge der Struktur somit allein durch die Distanzelemente gebildet. Die Distanzelemente sind demnach nicht in die Vorsprünge eingebettet, sondern bilden selbst die Vorsprünge und sind zu diesem Zweck auf der der zu behandelnden Oberfläche abgewandten Seite an die einen direkten Stromfluss verhindernde Schicht angefügt, d.h. mit ihr verbunden. Die Herstellung einer festen Verbindung zwischen den Distanzelementen und der Schicht des Dielektrikums kann dabei wiederum in üblicher Weise erfolgen, d.h. mechanisch in einer Gehäusestruktur, formschlüssig und/oder stoffschlüssig, insbesondere durch Kleben oder Schweißen. Auf der der Elektrode abgewandten Seite, d.h. auf der der zu behandelnden Oberfläche zugewandten Seite der Distanzelemente, bilden die Distanzelemente eine Anlagefläche an der zu behandelnden Oberfläche. In dieser Ausführungsform liegen die Distanzelemente somit während der Behandlung auf der zu behandelnden Oberfläche auf.

Eine solche Ausführungsform bietet den Vorteil, dass die Elektrodenanordnung besonders einfach hergestellt werden kann, da die Distanzelemente nach ihrer Herstellung, bspw. im Gießverfahren oder im 3D-Druckverfahren, lediglich an das Dielektrikum angefügt werden müssen.

Die Verbindung zwischen den Distanzelementen und der einen direkten Stromfluss verhindernden Schicht des Dielektrikums kann dabei vorteilhaft insbesondere eine lösbare Verbindung sein. Dies bietet den Vorteil, dass insbesondere bei der Behandlung von Wunden eine leichte Austauschbarkeit der mit der Wunde in Berührung gelangenden Distanzelemente ermöglicht wird, die wiederum als entfernbare Einmalteile verwendet oder wegen ihres geringen Volumens einfach sterilisiert werden können.

Vorteilhaft können die Distanzelemente dabei für eine Anlage auf der Haut eines Lebewesens ausgebildet sein.

In einer weiteren vorteilhaften Weiterbildung der Erfindung ist vorgesehen, dass die Struktur eine Gitterstruktur aus aneinander angrenzenden, die Vorsprünge bildenden Wänden ist, welche zahlreiche, die Lufträume ausbildende Kammern begrenzen. Die Gitterstruktur kann dabei vorteilhaft insbesondere einstückig mit der den direkten Stromfluss verhindernden Schicht des Dielektrikums ausgebildet sein. Alternativ kann die Gitterstruktur aber auch als separates Teil gefertigt und an die den direkten Stromfluss verhindernde Schicht des Dielektrikums angesetzt sein.

Die Distanzelemente können dabei vorteilhaft in die Wände der Gitterstruktur eingebettet, insbesondere vollständig in die Wände der Gitterstruktur eingebettet, sein.

Eine solche, aus der eingangs genannten DE 10 2015 117 715 A1 bekannte Gitterstruktur bietet in Kombination mit den Distanzelementen der erfindungsgemäßen Elektrodenanordnung die Vorteile, dass sie besonders flexibel und leicht ausgestaltet werden kann und durch die erfindungsgemäßen Distanzelemente zugleich zuverlässig eine Einhaltung des Abstands zwischen der zu behandelnden Oberfläche und der den direkten Stromfluss verhindernden Schicht - und damit die effektive Ausbildung des Plasmas -sicherstellt.

In einer weiteren vorteilhaften Weiterbildung der Erfindung ist vorgesehen, dass die Struktur aus vorstehenden, die Vorsprünge bildenden Noppen besteht, die in ihren Zwischenräumen die Lufträume ausbilden. Die Distanzelemente können dabei vorteilhaft in die Noppen eingebettet, insbesondere vollständig in die Noppen eingebettet sein.

Vorteilhaft können die Noppen dabei kreiszylindrisch, kegelförmig oder kegelstumpfförmig ausgebildet sein.

Derartige Ausführungsformen, bei denen die Struktur aus vorstehenden Noppen besteht, bieten die Vorteile, dass sie sich einfach und kostengünstig fertigen lassen und sich bei Verwendung eines hinreichend flexiblen Materials gut an die zu behandelnden Oberfläche anpassen, wobei die hinreichende Ausbildung der für die Plasmabildung notwendigen Lufträume durch die erfindungsgemäßen Distanzelemente sichergestellt werden kann.

In einer weiteren vorteilhaften Weiterbildung der Erfindung ist vorgesehen, dass die Vorsprünge der Struktur in der zuvor beschriebenen Weise vollständig durch die Distanzelemente gebildet sind und die Distanzelemente die Form von vorstehenden Noppen haben, die in ihren Zwischenräumen die Lufträume ausbilden. Die Noppen können dabei wiederum vorteilhaft kreiszylindrisch, kegelförmig oder kegelstumpfförmig ausgebildet sein.

Diese Weiterbildung der erfindungsgemäßen Elektrodenanordnung ähnelt somit der vorstehend beschriebenen Weiterbildung, wobei allerdings die Distanzelemente selbst die Form von vorstehenden Noppen haben. Demnach sind die Noppen aus dem zweiten Material gefertigt, dessen Flexibilität geringer ist als die Flexibilität des ersten Materials, aus dem das Dielektrikum der Elektrodenanordnung gefertigt ist.

Eine solche Ausführungsform, bei der die Distanzelemente die Form von vorstehenden Noppen haben, bietet den Vorteil, dass sie sich besonders einfach und kostengünstig herstellen lässt. Hierzu kann die Struktur mit den die Vorsprünge bildenden Distanzelementen in Form von vorstehenden Noppen insbesondere einstückig ausgebildet sein, sodass eine besonders einfache und kostengünstige Fertigung, bspw. im Gießverfahren oder im 3D-Druckverfahren, möglich ist.

In einer weiteren vorteilhaften Weiterbildung der Erfindung ist vorgesehen, dass die Distanzelemente jeweils aus einer umlaufenden Wandung gebildet sind.

Die Wandung umläuft das Distanzelement dabei auf den Seiten des Distanzelementes, die nicht die der zu behandelnden Oberfläche zugewandte Seite des Distanzelementes und nicht die der einen direkten Stromfluss verhindernden Schicht zugewandte Seite des Distanzelementes sind. Die umlaufende Wandung bildet auf diese Weise die seitliche Begrenzung eines von ihr umschlossenen Innenraums. Die umlaufende Wandung, aus der das jeweilige Distanzelement gebildet ist, kann dabei aus einer einzelnen umlaufenden Wand oder aus mehreren aneinander grenzenden Wänden bestehen. Die Höhe der Distanzelemente wird dabei durch die Höhe der umlaufenden Wandung bestimmt.

In einer weiteren vorteilhaften Weiterbildung der Erfindung ist vorgesehen, dass die umlaufenden Wandungen der Distanzelemente jeweils einen Luftraum umschließen.

Die Distanzelemente können demnach so in der Elektrodenanordnung angeordnet werden, dass sich die Lufträume für die Ausbildung des Plasmas innerhalb des durch die umlaufende Wandung des jeweiligen Distanzelementes begrenzten Innenraums ausbilden.

Die umlaufenden Wandungen der Distanzelemente können vorteilhaft jeweils einen viereckigen, insbesondere rechteckigen oder quadratischen, Querschnitt aufweisen. Die umlaufenden Wandungen der Distanzelemente können vorteilhaft aber auch einen runden oder ovalen Querschnitt aufweisen. Die umlaufenden Wandungen der Distanzelemente können vorteilhaft aber auch einen vieleckigen, insbesondere einen wabenförmigen, Querschnitt aufweisen.

Die Materialstärke der Wandung kann vorteilhaft weniger als 20 %, insbesondere weniger als 10 %, der größten Breite des von der Wandung umschlossenen Raums ausmachen. Die Materialstärke der Wandung kann dabei vorteilhaft bspw. zwischen 0,1 mm und 1,0 mm betragen.

Derartige Ausführungsformen der erfindungsgemäßen Elektrodenanordnung mit Distanzelementen, die jeweils aus einer umlaufenden Wandung gebildet sind, bieten den erfindungsgemäßen Vorteil, dass die Distanzelemente aufgrund ihrer Form eine besonders hohe Stabilität aufweisen. Dadurch können die Distanzelemente mit geringem Materialaufwand gefertigt werden. Sie weisen zudem eine besonders kleine Querschnittsfläche auf. Hierdurch wird wiederum vorteilhaft erreicht, dass die Vorsprünge der Struktur nur eine kleine Fläche auf der zu behandelnden Oberfläche einnehmen und ein großer Teil der zu behandelnden Oberfläche für die Ausbildung der Lufträume und damit für die Ausbildung des Plasmas zur Verfügung steht. Dadurch ist vorteilhaft eine besonders effektive Plasmabehandlung möglich.

In einer weiteren vorteilhaften Weiterbildung der Erfindung ist vorgesehen, dass die Distanzelemente und/oder die Vorsprünge der Struktur eine einheitliche Höhe aufweisen.

Dadurch kann vorteilhaft eine besonders gleichmäßige Ausbildung des Plasmas erreicht werden. Darüber hinaus ist es auf diese Weise möglich, dass zwischen den Vorsprüngen Kammern gebildet werden, die seitlich geschlossen sind und einen abgeschlossenen Luftraum bilden, wenn die Elektrodenanordnung auf der zu behandelnden Oberfläche aufliegt. Untersuchungen zeigen, dass auch in derartigen abgeschlossenen Kammern ein geeignetes Plasma ausgebildet werden kann.

Die Erfindung soll im Folgenden anhand der in den beigefügten Zeichnungen schematisch dargestellten Ausführungsbeispiele näher erläutert werden. Es zeigen:
- Figur 1a): - eine Ansicht auf eine Oberseite einer ersten Ausführungsform einer Elektrodenanordnung;
- Figur 1b): - einen Vertikalschnitt entlang der Linie A-A in Figur 1a);
- Figur 1c): - eine Detailansicht des Ausschnitts A in Figur 1b);
- Figur 2a): - eine Ansicht auf eine Auflageseite der ersten Ausführungsform einer Elektrodenanordnung;
- Figur 2b): - einen Vertikalschnitt entlang der Linie B-B in Figur 2a);
- Figur 3a): - einen Horizontalschnitt entlang der Linie C-C in Figur 1b);
- Figur 3b): - einen Horizontalschnitt entlang der Linie D-D in Figur 1b);
- Figur 4a): - eine Ansicht auf eine Oberseite einer zweiten Ausführungsform einer Elektrodenanordnung;
- Figur 4b): - einen Vertikalschnitt entlang der Linie A-A in Figur 4a);
- Figur 4c): - eine Detailansicht des Ausschnitts A in Figur 4b);
- Figur 5a): - eine Ansicht auf eine Auflageseite der zweiten Ausführungsform einer Elektrodenanordnung;
- Figur 5b): - einen Vertikalschnitt entlang der Linie B-B in Figur 5a);
- Figur 6a): - einen Horizontalschnitt entlang der Linie C-C in Figur 4b);
- Figur 6b): - einen Horizontalschnitt entlang der Linie D-D in Figur 4b);
- Figur 7a): - eine Ansicht auf eine Oberseite einer dritten Ausführungsform einer Elektrodenanordnung;
- Figur 7b): - einen Vertikalschnitt entlang der Linie A-A in Figur 7a);
- Figur 7c): - eine Detailansicht des Ausschnitts A in Figur 7b);
- Figur 8a): - eine Ansicht auf eine Auflageseite der dritten Ausführungsform einer Elektrodenanordnung;
- Figur 8b): - einen Vertikalschnitt entlang der Linie B-B in Figur 8a).

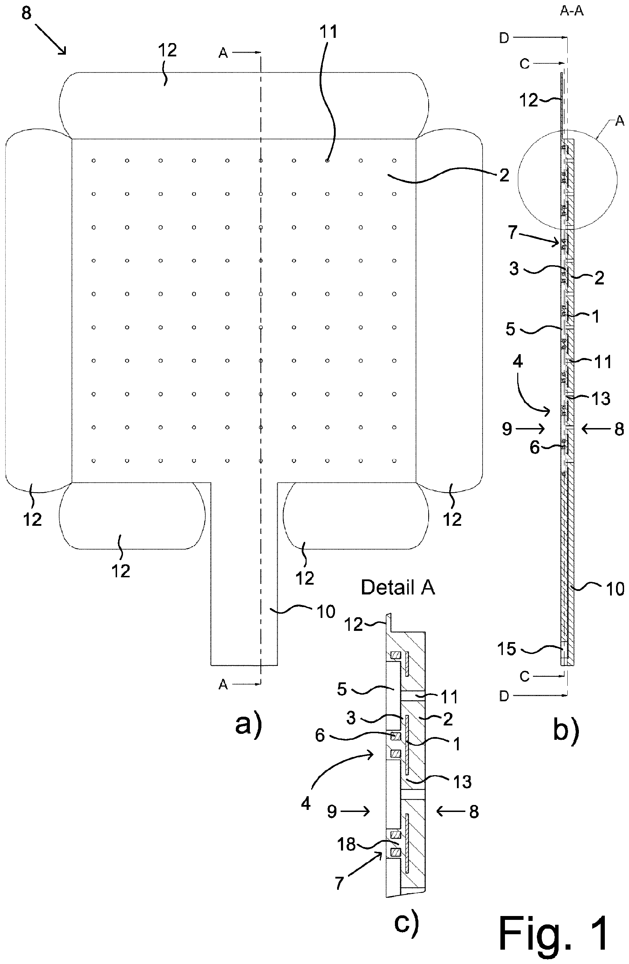

Das in Figur 1a) dargestellte Ausführungsbeispiel zeigt eine Oberseite 8, d.h. eine von der zu behandelnden Oberfläche abgewandte Seite, eines ersten Ausführungsbeispiels einer Elektrodenanordnung für eine dielektrisch behinderte Plasmabehandlung einer Oberfläche eines Körpers. Zu erkennen ist die Oberseite 8 eines Dielektrikums 2 mit einem im Wesentlichen quadratischen Querschnitt. Auf einer Seite erstreckt sich die Elektrodenanordnung in einen stegförmigen Ansatz 10. Das Dielektrikum 2 weist eine Vielzahl von Durchgangsöffnungen 11 auf, welche bspw. die Abführung eines Fluids, insbesondere einer Flüssigkeit, von der zu behandelnden Oberfläche ermöglichen. Die Elektrodenanordnung weist darüber hinaus mehrere flügelförmige Abschnitte 12 auf, die mit einer geringen Dicke besonders flexibel und auf ihrer Unterseite klebend ausgebildet sind, um die Befestigung der Elektrodenanordnung auf der Haut eines Lebewesens, gegebenenfalls um eine Wunde herum, zu ermöglichen.

Das Dielektrikum 2 ist aus einem besonders flexiblen Silikon gefertigt, das eine geringe Härte von lediglich 20 Shore aufweist. Dadurch weist das Dielektrikum 2 nur eine sehr geringe Steifigkeit auf, kann sich aber in seiner Form sehr gut an die zu behandelnde Oberfläche anpassen.

Die Figur 1b) zeigt einen Vertikalschnitt durch die Elektrodenanordnung entlang der Linie A-A in Figur 1a). Details können dabei der Detailansicht des Ausschnitts A in Figur 1c) entnommen werden. Zu erkennen ist eine flexible flächige Elektrode 1, die allseitig von dem Dielektrikum 2 umgeben ist. Das Dielektrikum 2 bildet insbesondere eine einen direkten oder galvanischen Stromfluss verhindernde Schicht 3 aus, die sich entlang der gesamten Fläche der Elektrode 1 erstreckt und die Elektrode so vollständig von der zu behandelnden Oberfläche abschirmt. Dadurch wird ein direkter oder galvanischer Stromfluss von der Elektrode 1 zu der zu behandelnden Oberfläche verhindert.

Das Dielektrikum 2 kann über eine Struktur 4 mit Vorsprüngen 7 auf der zu behandelnden Oberfläche aufliegen und bildet dabei zwischen den Vorsprüngen 7 Lufträume 5 für die Ausbildung des Plasmas aus. Die Lufträume 5 weisen einen bodenseitigen Abschluss durch die den direkten Stromfluss verhindernde Schicht 3 des Dielektrikums 2 und eine zur behandelnden Oberfläche offene Seite auf, sind also auf einer Auflageseite 9 der Elektrodenanordnung offen.

Zu erkennen ist des Weiteren, dass nicht nur das Dielektrikum 2 Durchgangsöffnungen 11 hat, sondern auch die Elektrode 1 Durchgangsöffnungen 13 aufweist. Die Durchgangsöffnungen 11 des Dielektrikums fluchten dabei mit den Durchgangsöffnungen 13 der Elektrode und den Lufträumen 5 zwischen den Vorsprüngen 7 der Struktur 4, sodass durch die Durchgangsöffnungen 11, 13 die Abführung eines Fluids, insbesondere einer Flüssigkeit, von der zu behandelnden Oberfläche möglich ist.

Des Weiteren lassen die Figuren 1b) und 1c) erkennen, dass die Struktur 4 eine Vielzahl von Distanzelementen 6 aufweist, die in diesem Ausführungsbeispiel aus einem stabilen Kunststoff gefertigt sind. Die Flexibilität dieses stabilen Kunststoffs ist dabei geringer als die Flexibilität des Silikons, aus dem das Dielektrikum 2 gefertigt ist.

Die Distanzelemente 6 sind in diesem Ausführungsbeispiel jeweils aus einer umlaufenden Wandung gebildet, die jeweils einen Luftraum 5 umschließt. Die umlaufenden Wandungen der Distanzelemente 6 weisen dabei einen rechteckigen Querschnitt auf.

Die Figuren 1b) und 1c) lassen außerdem erkennen, dass die Vorsprünge 7 der Struktur 4 teilweise durch die Distanzelemente 6, nämlich aus den Distanzelementen 6 und dem Dielektrikum 2, gebildet sind. Die Distanzelemente 6 sind dabei vollständig in das sie umgebende Dielektrikum 2 eingebettet und bilden so eine Verstärkung der Vorsprünge 7. Das die Distanzelemente 6 umgebende Dielektrikum 2 ist einstückig mit der den direkten Stromfluss verhindernden Schicht 3 des Dielektrikums 2 ausgebildet. Dies ermöglicht eine besonders einfache und kostengünstige Fertigung des Dielektrikums 2 im Gießverfahren, wobei die Distanzelemente 6 in das Dielektrikum 2 eingegossen werden.

Die Distanzelemente 6 sind über Verbindungsabschnitte 18, die eine geringere Biegesteifigkeit und eine geringere Torsionssteifigkeit aufweisen als die Distanzelemente 6, miteinander verbunden. Die Verbindungsabschnitte 18 zwischen den Distanzelementen 6 sind dabei durch das Dielektrikum 2, nämlich durch die einen direkten Stromfluss verhindernde Schicht 3 des Dielektrikums 2, gebildet. Da das besonders flexible Silikon des Dielektrikums 2 eine größere Flexibilität aufweist als der stabile Kunststoff der Distanzelemente 6, kann auf diese Weise besonders einfach erreicht werden, dass die Verbindungsabschnitte 18 eine geringere Biege- und Torsionssteifigkeit aufweisen als die Distanzelemente 6. Die Struktur 4 weist dadurch in der Fläche eine deutlich höhere Flexibilität aufweist als in der Höhe.

Die Elektrodenanordnung liegt während der Behandlung mit dem sehr weichen und flexiblen Dielektrikum 2 auf der zu behandelnden Hautoberfläche auf.

Sowohl die Distanzelemente 6 als auch die Vorsprünge 7 der Struktur 4 weisen in diesem Ausführungsbeispiel eine einheitliche Höhe auf.

Deutlich wird zudem, dass sich die Elektrode 1 in den stegförmigen Ansatz 10 hineinerstreckt. Das Dielektrikum 2 weist im Bereich des stegförmigen Ansatzes 10 Aussparungen 15 auf, über welche die Kontaktierung der Elektrode 1 zur Zuführung einer für die Plasmaerzeugung benötigten Hochspannung, die vorzugsweise als Wechselspannung verwendet wird, erfolgen kann.

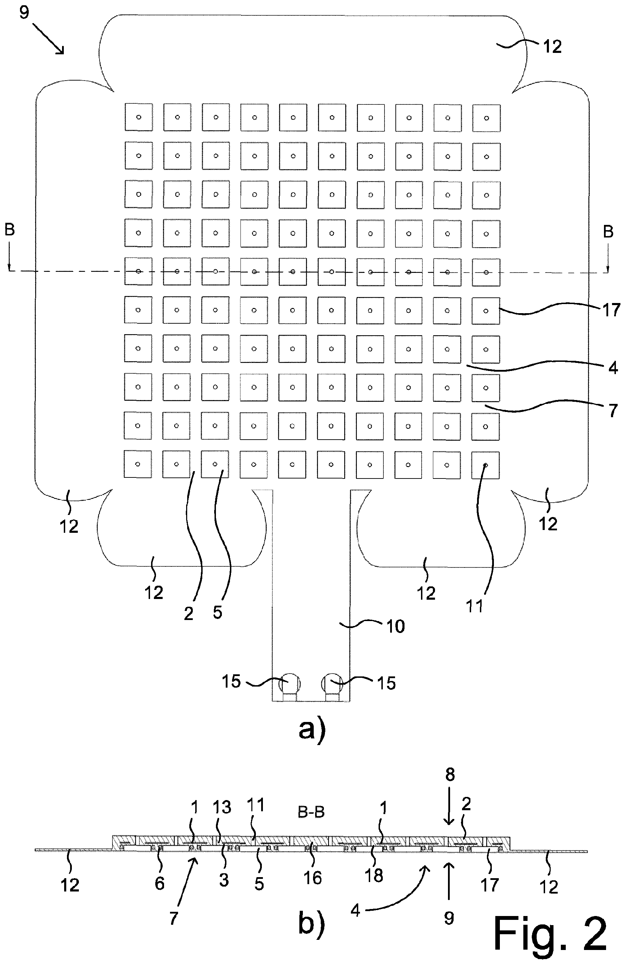

Die Figur 2a) zeigt die Auflageseite 9 der Elektrodenanordnung des ersten Ausführungsbeispiels. Zu erkennen ist insbesondere, dass die Struktur 4 als Gitterstruktur aus aneinander angrenzenden, die Vorsprünge 7 bildenden Wänden ausgebildet ist, wobei die Wände zahlreiche, die Lufträume 5 ausbildende Kammern 17 begrenzen. Die Wände der Gitterstruktur bilden auf ihrer der zu behandelnden Oberfläche zugewandten Seite dabei eine Auflagefläche an der zu behandelnden Oberfläche.

Der stegförmige Ansatz 10 weist zwei Aussparungen 15 für die Kontaktierung jeweils einer Elektrode 1 der Elektrodenanordnung auf, da die Elektrodenanordnung insgesamt zwei Elektroden 1 aufweist, wie im Folgenden noch deutlich wird.

Die Figur 2b) zeigt einen Vertikalschnitt durch die Elektrodenanordnung entlang der Linie B-B in Figur 2a). Zu erkennen ist dabei, dass die Elektrodenanordnung zwei Elektroden 1 aufweist, die allseitig von dem Dielektrikum 2 umgeben sind. Das Dielektrikum 2 weist daher einen mittleren Bereich 16 auf, in den sich die Elektroden 1 nicht erstrecken.

Die Distanzelemente 6 sind in die aus dem Dielektrikum 2 gebildeten Wände, welche die Vorsprünge 7 bilden, eingebettet.

Im Übrigen kann bezüglich der Figuren 2a) und 2b) auf die obigen Ausführungen zu den Figuren 1a) bis 1c) verwiesen werden.

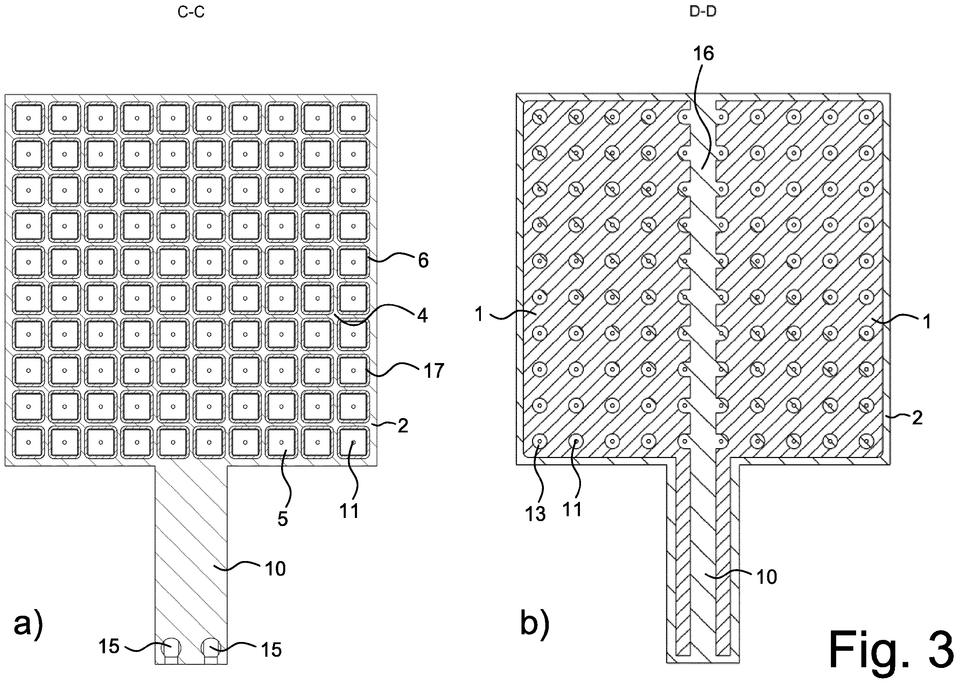

Die Figur 3a) zeigt einen Horizontalschnitt durch die Elektrodenanordnung des ersten Ausführungsbeispiels entlang der Linie C-C in der Figur 1b). Erkennbar wird dabei, dass die Struktur 4 eine Vielzahl von Distanzelementen 6 aufweist, die zusammen mit dem Dielektrikum 2 die Vorsprünge 7 bilden. Die Distanzelemente 6 sind jeweils aus einer umlaufenden Wandung gebildet und umschließen jeweils einen Luftraum 5, der in einer Kammer 17, die durch die Wände der Gitterstruktur 4 begrenzt wird, ausgebildet ist. Die Wandung der Distanzelemente 6 umschließt dabei demnach die durch die Wände der Gitterstruktur 4 gebildete Kammer 17.

Deutlich erkennbar wird, dass jedes Distanzelement 6 jeweils einen viereckigen Querschnitt aufweist.

Die Figur 3b) zeigt einen Horizontalschnitt durch die Elektrodenanordnung des ersten Ausführungsbeispiels entlang der Line D-D der Figur 1b). Erkennbar wird dabei, dass die Elektrodenanordnung zwei Elektroden 1 und einen mittleren Bereich 16 des Dielektrikums 2, in den sich die Elektroden 1 nicht erstrecken, aufweist. Die Elektroden 1 erstrecken sich, um ihre Kontaktierung zu ermöglichen, jeweils in den stegförmigen Ansatz 10.

Erkennbar ist auch, dass die Durchgangsöffnungen 11 des Dielektrikums 2 einen kleineren Durchmesser aufweisen als die Durchgangsöffnungen 13 der Elektrode 1. Dadurch kann sichergestellt werden, dass das Dielektrikum 2 die Elektrode 1 allseitig umgibt und die Elektrode 1 somit vollständig von der zu behandelnden Oberfläche abschirmt. Zum gleichen Zweck dient, dass sich das Dielektrikum 2 an seinen seitlichen Rändern über die Fläche der Elektrode 1 hinaus erstreckt, sodass auch an den seitlichen Rändern der Elektrodenanordnung die Elektrode 1 vollständig von dem Dielektrikum 2 umgeben wird.

Im Übrigen kann bezüglich der Figuren 3a) und 3b) auf die Ausführungen zu den Figuren 1 und 2 verwiesen werden.

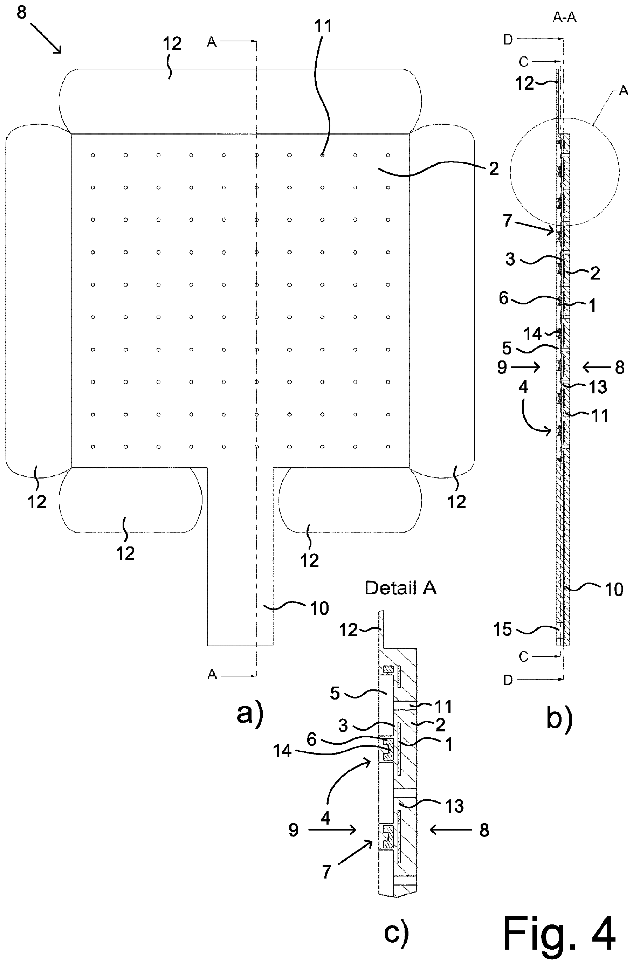

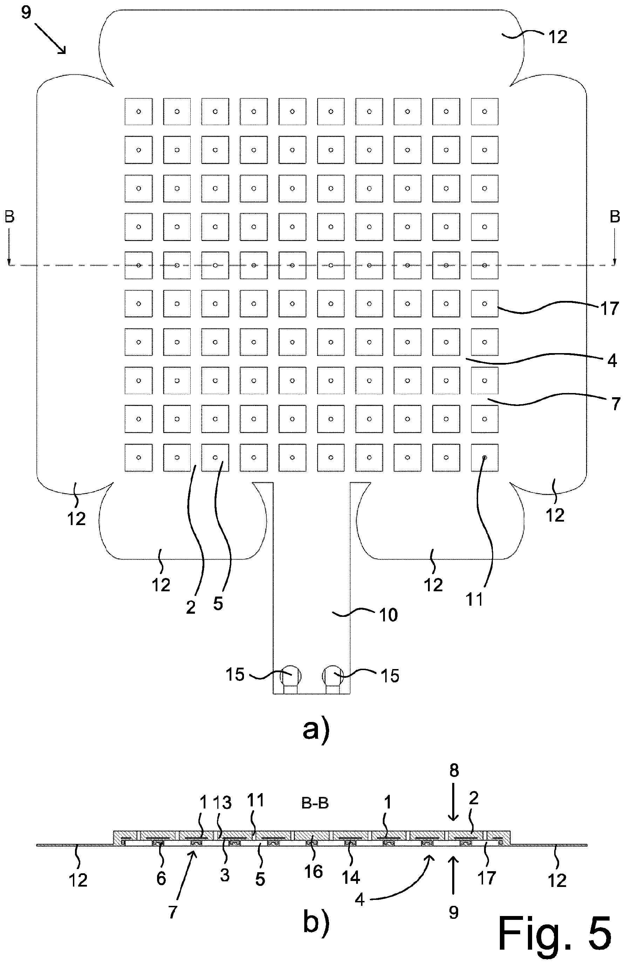

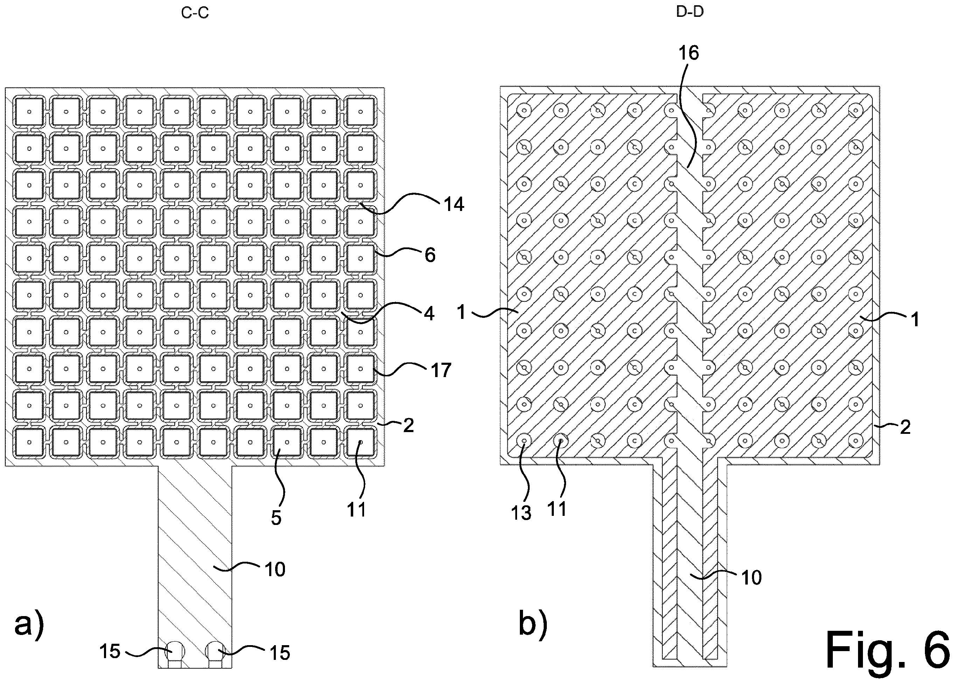

Die Figuren 4, 5 und 6 zeigen in einer mit den Figuren 1, 2 bzw. 3 übereinstimmenden Darstellungsform ein zweites Ausführungsbeispiel einer erfindungsgemäßen Elektrodenanordnung.

Dieses zweite Ausführungsbeispiel der erfindungsgemäßen Elektrodenanordnung unterscheidet sich, wie insbesondere in den Figuren 4b), 4c), 5b) und 6a) erkennbar ist, dadurch von dem ersten Ausführungsbeispiel, dass die Distanzelemente 6 durch Verbindungsstege 14 miteinander verbunden sind. Die Distanzelemente 6 bilden auf diese Weise ein Distanzgitter aus. Dadurch kann eine besonders hohe Stabilität der Distanzelemente 6 erreicht werden, die besonders zuverlässig sicherstellt, dass der notwendige Abstand zwischen der zu behandelnden Oberfläche und der einen direkten Stromfluss verhindernden Schicht 3 während der Behandlung eingehalten wird.

Das aus den Distanzelementen 6 mit Hilfe der Verbindungsstege 14 gebildete Distanzgitter ist in diesem Ausführungsbeispiel einstückig ausgebildet und kann daher besonders einfach im Gießverfahren gefertigt werden.

Im Übrigen kann bezüglich des in den Figuren 4 bis 6 gezeigten zweiten Ausführungsbeispiels auf die Ausführungen zu dem ersten Ausführungsbeispiel verwiesen werden.

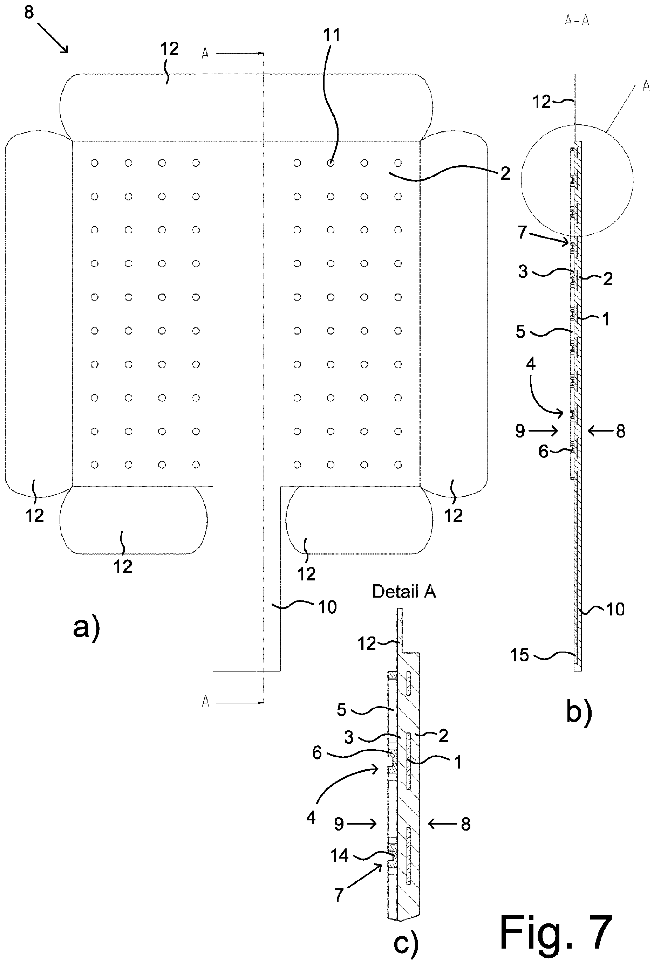

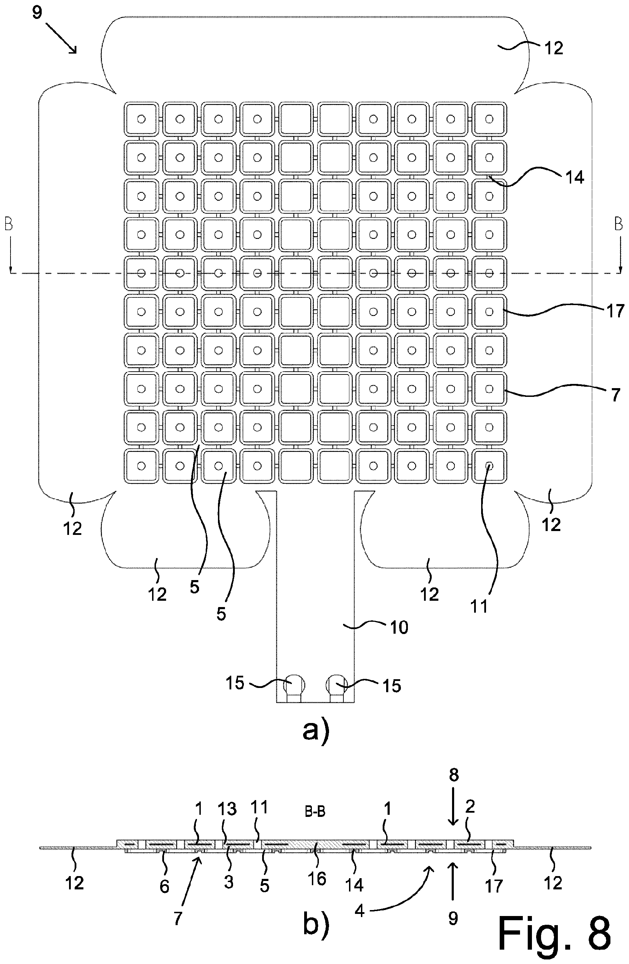

Die Figuren 7 und 8 zeigen in einer mit den Figuren 1 bzw. 2 sowie den Figuren 4 bzw. 5 übereinstimmenden Darstellungsform ein drittes Ausführungsbeispiel einer erfindungsgemäßen Elektrodenanordnung.

Dieses dritte Ausführungsbeispiel der erfindungsgemäßen Elektrodenanordnung unterscheidet sich, wie in den Figuren 7b), 7c), 8a) und 8b) erkennbar ist, dadurch von dem ersten und zweiten Ausführungsbeispiel, dass die Vorsprünge 7 der Struktur 4 allein durch die Distanzelemente 6 gebildet sind. Die Distanzelemente 6 sind dabei auf ihrer der Elektrode 1 zugewandten Seite mit der einen direkten Stromfluss verhindernden Schicht 3 des Dielektrikums 2 verbunden. Auf der entgegensetzten Seite der Distanzelemente 6, welche der zu behandelnden Oberfläche zugewandt ist, bilden die Distanzelemente 6 eine Anlagefläche an der zu behandelnden Oberfläche. Die Distanzelemente 6 weisen dabei eine einheitliche Höhe auf, sind für eine Anlage auf der Haut eines Lebewesens ausgebildet und liegen während der Behandlung auf der Haut auf.

Die Distanzelemente 6 sind im Gegensatz zum ersten und zweiten Ausführungsbeispiel in dem in den Figuren 7 und 8 gezeigten dritten Ausführungsbeispiel somit nicht in die Vorsprünge 7 eingebettet, sondern die Distanzelemente 6 bilden selbst die vollständigen Vorsprünge 7. Zu diesem Zweck sind die Distanzelemente 6 und die einen direkten Stromfluss verhindernde Schicht 3 des Dielektrikums 2 durch Kleben aneinandergefügt, d. h. durch eine stoffschlüssige Verbindung fest miteinander verbunden.

Die Distanzelemente 6 sind jeweils aus einer umlaufenden Wandung gebildet, die einen im Wesentlichen quadratischen Querschnitt aufweist und einen Luftraum 5 umschließt. Darüber hinaus sind im Gegensatz zum ersten und zweiten Ausführungsbeispiel in diesem dritten Ausführungsbeispiel auch in den Zwischenräumen zwischen den verschiedenen Distanzelementen 6 Lufträume 5 vorhanden, in denen sich das Plasma ausbilden kann.

In Übereinstimmung mit dem zweiten Ausführungsbeispiel sind die Distanzelemente 6 auch in dem dritten Ausführungsbeispiel durch Verbindungsstege 14 miteinander verbunden. Die Distanzelemente 6 bilden auf diese Weise ein einstückig ausgeführtes Distanzgitter aus, das besonders einfach im Gießverfahren gefertigt werden kann.

Im Übrigen kann bezüglich des in den Figuren 7 und 8 gezeigten dritten Ausführungsbeispiels auf die Ausführungen zu dem ersten und zweiten Ausführungsbeispiel verwiesen werden.

## Patentansprüche

1. Elektrodenanordnung für eine dielektrisch behinderte Plasmabehandlung einer als Gegenelektrode verwendeten Oberfläche eines elektrisch leitenden Körpers, mit mindestens einer flexiblen flächigen Elektrode (1) und einem Dielektrikum (2) aus einem flächigen flexiblen ersten Material, das mit einer einen direkten Stromfluss verhindernden Schicht (3) die Elektrode (1) von der zu behandelnden Oberfläche abschirmt, wobei das Dielektrikum (2) über eine Struktur (4) mit Vorsprüngen (7) auf der zu behandelnden Oberfläche aufliegen kann und dabei zwischen den Vorsprüngen (7) Lufträume (5) für die Ausbildung des Plasmas ausgebildet sind, die eine zur behandelnden Oberfläche offene Seite und einen bodenseitigen Abschluss durch die den direkten Stromfluss verhindernde Schicht (3) des Dielektrikums (2) aufweisen, **dadurch gekennzeichnet, dass** die Struktur (4) eine Vielzahl von Distanzelementen (6) aus einem zweiten Material, dessen Flexibilität geringer ist als die Flexibilität des ersten Materials, aufweist, wobei die Vorsprünge (7) der Struktur (4) teilweise oder vollständig durch die Distanzelemente (6) gebildet sind.

2. Elektrodenanordnung nach Anspruch 1, **dadurch gekennzeichnet, dass** die Distanzelemente (6) über Verbindungsabschnitte (18), die eine geringere Biegesteifigkeit und/oder eine geringere Torsionssteifigkeit aufweisen als die Distanzelemente (6), miteinander verbunden sind.

3. Elektrodenanordnung nach Anspruch 1 oder 2, **dadurch gekennzeichnet, dass** die Distanzelemente (6) durch Verbindungsstege (14) miteinander verbunden sind und auf diese Weise ein Distanzgitter ausbilden.

4. Elektrodenanordnung nach Anspruch 3, **dadurch gekennzeichnet, dass** das Distanzgitter einstückig ausgebildet ist.

5. Elektrodenanordnung nach einem der vorhergehenden Ansprüche, **dadurch gekennzeichnet, dass** die Distanzelemente (6) im Inneren der Vorsprünge (7) eingebettet sind, insbesondere vollständig im Inneren der Vorsprünge (7) eingebettet sind, um die Vorsprünge (7) zu verstärken.

6. Elektrodenanordnung nach Anspruch 5, **dadurch gekennzeichnet, dass** die Vorsprünge (7) der Struktur (4) aus den Distanzelementen (6) und dem Dielektrikum (2) gebildet sind, wobei die Distanzelemente (6) in das die Distanzelemente (6) umgebende Dielektrikum (2) eingebettet, insbesondere vollständig eingebettet, sind.

7. Elektrodenanordnung nach Anspruch 6, **dadurch gekennzeichnet, dass** das die Distanzelemente (6) umgebende Dielektrikum (2) einstückig mit der den direkten Stromfluss verhindernden Schicht (3) des Dielektrikums (2) ausgebildet ist.

8. Elektrodenanordnung nach einem der Ansprüche 1 bis 4, **dadurch gekennzeichnet, dass** die Vorsprünge (7) der Struktur (4) vollständig durch die Distanzelemente (6) gebildet sind, wobei die Distanzelemente (6) auf ihrer der Elektrode (1) zugewandten Seite mit der einen direkten Stromfluss verhindernden Schicht (3) des Dielektrikums (2) verbunden sind und auf ihrer der zu behandelnden Oberfläche zugewandten Seite eine Anlagefläche an der zu behandelnden Oberfläche bilden.

9. Elektrodenanordnung nach einem der vorhergehenden Ansprüche, **dadurch gekennzeichnet, dass** die Struktur (4) eine Gitterstruktur aus aneinander angrenzenden, die Vorsprünge (7) bildenden Wänden ist, welche zahlreiche, die Lufträume (5) ausbildende Kammern (17) begrenzen.

10. Elektrodenanordnung nach einem der Ansprüche 1 bis 7, **dadurch gekennzeichnet, dass** die Struktur (4) aus vorstehenden, die Vorsprünge (7) bildenden Noppen besteht, die in ihren Zwischenräumen die Lufträume (5) ausbilden.

11. Elektrodenanordnung nach Anspruch 8, **dadurch gekennzeichnet, dass** die Distanzelemente (6) die Form von vorstehenden Noppen haben, die in ihren Zwischenräumen die Lufträume (5) ausbilden.

12. Elektrodenanordnung nach Anspruch 10 oder 11, **dadurch gekennzeichnet, dass** die Noppen kreiszylindrisch, kegelförmig oder kegelstumpfförmig ausgebildet sind.

13. Elektrodenanordnung nach einem der Ansprüche 1 bis 10 oder 12, **dadurch gekennzeichnet, dass** die Distanzelemente (6) jeweils aus einer umlaufenden Wandung gebildet sind.

14. Elektrodenanordnung nach Anspruch 13, **dadurch gekennzeichnet, dass** die umlaufenden Wandungen der Distanzelemente (6) jeweils einen Luftraum (5) umschließen.

15. Elektrodenanordnung nach Anspruch 13 oder 14, **dadurch gekennzeichnet, dass** die umlaufenden Wandungen jeweils einen viereckigen, insbesondere rechteckigen oder quadratischen, runden, ovalen oder vieleckigen, insbesondere wabenförmigen, Querschnitt aufweisen.

16. Elektrodenanordnung nach einem der vorhergehenden Ansprüche, **dadurch gekennzeichnet, dass** die Distanzelemente (6) und/oder die Vorsprünge (7) der Struktur (4) eine einheitliche Höhe aufweisen.

## Claims

1. An electrode arrangement for a dielectrically hindered plasma treatment of a surface of an electroconductive body used as a counter electrode with at least one flexible, planar electrode (1) and a dielectric (2) made of a planar, flexible first material that shields the electrode (1) from the surface to be treated by way of a layer (2) that prevents a direct current, wherein the dielectric (2) can rest on the surface to be treated via a structure (4) with projections (7) and there are air spaces (5) between the projections (7) for forming the plasma, said air spaces having a side open towards the surface to be treated and a bottom-side closure formed by the layer (3) of the dielectric (2) that prevents the direct current, **characterised in that** the structure (4) comprises a number of spacer elements (6) made of a second material, the flexibility of which is lower than the flexibility of the first material, the projections (7) of the structure (4) being partially or fully formed by the spacer elements (6).

2. The electrode arrangement according to claim 1, **characterised in that** the spacer elements (6) are connected to each other via connecting sections (18) that have a lower bending stiffness and/or a lower torsional stiffness than the spacer elements (6).

3. The electrode arrangement according to claim 1 or 2, **characterised in that** the spacer elements (6) are connected to each other by connecting webs (14), thereby forming a spacer grid.

4. The electrode arrangement according to claim 3, **characterised in that** the spacer grid is designed as a single piece.

5. The electrode arrangement according to one of the preceding claims, **characterised in that** the spacer elements (6) are embedded inside the projections (7), in particular completely embedded inside the projections (7), so as to reinforce the projections (7).

6. The electrode arrangement according to claim 5, **characterised in that** the projections (7) of the structure (4) are formed of the spacer elements (6) and the dielectric (2), the spacer elements (6) being embedded, in particular completely embedded, in the dielectric (2) that surrounds the spacer elements (6).

7. The electrode arrangement according to claim 6, **characterised in that** the dielectric (2) that surrounds the spacer elements (6) is designed as a single piece with the layer (3) of the dielectric (2) that prevents the direct current.

8. The electrode arrangement according to one of the claims 1 to 4, **characterised in that** the projections (7) of the structure (4) are formed entirely by the spacer elements (6), the spacer elements (6) being connected on their side facing the electrode (1) with the layer (3) of the dielectric (2) that prevents a direct current and forming, on their side facing the surface to be treated, a contact surface on the surface to be treated.

9. The electrode arrangement according to one of the preceding claims, **characterised in that** the structure (4) is a lattice structure of adjacent walls forming the projections (7) which delimit numerous chambers (17) that form the air spaces (5).

10. The electrode arrangement according to one of the claims 1 to 7, **characterised in that** the structure (4) consists of protruding studs forming the projections (7), which form the air spaces (5) in their intermediate spaces.

11. The electrode arrangement according to claim 8, **characterised in that** the spacer elements (6) are in the shape of protruding studs, which form the air spaces (5) in their intermediate spaces.

12. The electrode arrangement according to claim 10 or 11, **characterised in that** the studs are designed to be circular-cylindrical, conical or frustoconical in shape.

13. The electrode arrangement according to one of the claims 1 to 10 or 12, **characterised in that** the spacer elements (6) are each formed by a circumferential wall.

14. The electrode arrangement according to claim 13, **characterised in that**, in each case, the circumferential wall of the spacer elements (6) encloses an air space (5).

15. The electrode arrangement according to claim 13 or 14, **characterised in that** the circumferential walls each have a quadrangular, in particular rectangular or square, round, oval or polygonal, in particular honeycomb, cross-section.

16. The electrode arrangement according to one of the preceding claims, **characterised in that** the spacer elements (6) and/or the projections (7) of the structure (4) have a uniform height.

## Revendications

1. Ensemble d'électrode pour un traitement d'une surface d'un corps électriquement conducteur avec un plasma à barrière diélectrique, ladite surface faisant office de contre-électrode, comprenant au moins une électrode surfacique flexible (1) et un diélectrique (2) constitué d'un premier matériau surfacique flexible, qui protège l'électrode (1) vis-à-vis de la surface à traiter par une couche (3) empêchant une circulation directe du courant, le diélectrique (2) pouvant reposer sur la surface à traiter par l'intermédiaire d'une structure (4) ayant des protubérances (7), et des espaces d'air (5) pour réaliser le plasma étant ménagés entre les protubérances (7), qui présentent un côté ouvert vers la surface à traiter et une terminaison côté fond formée par la couche (3) du diélectrique (2) empêchant la circulation directe du courant,
**caractérisé en ce que** la structure (4) présente une pluralité d'éléments d'écartement (6) constitués d'un deuxième matériau dont la flexibilité est inférieure à la flexibilité du premier matériau, les protubérances (7) de la structure (4) étant formées partiellement ou complètement par les éléments d'écartement (6).

2. Ensemble d'électrode selon la revendication 1,
**caractérisé en ce que** les éléments d'écartement (6) sont reliés entre eux par des portions de liaison (18) qui présentent une rigidité en flexion et/ou une rigidité en torsion inférieure à celle des éléments d'écartement (6).

3. Ensemble d'électrode selon la revendication 1 ou 2,
**caractérisé en ce que** les éléments d'écartement (6) sont reliés entre eux par des entretoises de liaison (14) et forment ainsi une grille d'écartement.

4. Ensemble d'électrode selon la revendication 3,
**caractérisé en ce que** la grille d'écartement est réalisée d'une seule pièce.

5. Ensemble d'électrode selon l'une des revendications précédentes, **caractérisé en ce que** les éléments d'écartement (6) sont noyés à l'intérieur des protubérances (7), en particulier complètement noyés à l'intérieur des protubérances (7), afin de renforcer les protubérances (7).

6. Ensemble d'électrode selon la revendication 5,
**caractérisé en ce que** les protubérances (7) de la structure (4) sont formées par les éléments d'écartement (6) et par le diélectrique (2), les éléments d'écartement (6) étant noyés, en particulier complètement noyés, dans le diélectrique (2) entourant les éléments d'écartement (6).

7. Ensemble d'électrode selon la revendication 6,
**caractérisé en ce que** le diélectrique (2) entourant les éléments d'écartement (6) est réalisé d'une seule pièce avec la couche (3) du diélectrique (2) empêchant la circulation directe du courant.

8. Ensemble d'électrode selon l'une des revendications 1 à 4,
**caractérisé en ce que** les protubérances (7) de la structure (4) sont formées complètement par les éléments d'écartement (6), les éléments d'écartement (6) étant reliés, sur leur côté tourné vers l'électrode (1), à la couche (3) du diélectrique (2) empêchant la circulation directe du courant et formant, sur leur côté tourné vers la surface à traiter, une surface d'appui contre la surface à traiter.

9. Ensemble d'électrode selon l'une des revendications précédentes, **caractérisé en ce que** la structure (4) est une structure en grille constituée de parois adjacentes qui forment les protubérances (7) et qui délimitent de nombreux compartiments (17) formant les espaces d'air (5).

10. Ensemble d'électrode selon l'une des revendications 1 à 7,
**caractérisé en ce que** la structure (4) est constituée de plots en saillie formant les protubérances (7), qui forment dans leurs espaces intermédiaires les espaces d'air (5).

11. Ensemble d'électrode selon la revendication 8,
**caractérisé en ce que** les éléments d'écartement (6) ont la forme de plots en saillie qui forment dans leurs espaces intermédiaires les espaces d'air (5).

12. Ensemble d'électrode selon la revendication 10 ou 11,
**caractérisé en ce que** les plots sont réalisés en forme de cylindre circulaire, de cône ou de tronc de cône.

13. Ensemble d'électrode selon l'une des revendications 1 à 10 ou 12, **caractérisé en ce que** les éléments d'écartement (6) sont formés chacun d'une paroi périphérique.

14. Ensemble d'électrode selon la revendication 13,
**caractérisé en ce que** les parois périphériques des éléments d'écartement (6) entourent chacune un espace d'air (5).

15. Ensemble d'électrode selon la revendication 13 ou 14,
**caractérisé en ce que** les parois périphériques présentent chacune une section transversale quadrangulaire, en particulier rectangulaire ou carrée, ronde, ovale ou polygonale, en particulier en forme de nid d'abeilles.

16. Ensemble d'électrode selon l'une des revendications précédentes,
**caractérisé en ce que** les éléments d'écartement (6) et/ou les protubérances (7) de la structure (4) présentent une hauteur uniforme.
